# EUROPÄISCHE PATENTSCHRIFT

(11) **EP 1 452 583 B1**
(45) Veröffentlichungstag und Bekanntmachung des Hinweises auf die Patenterteilung: **03.05.2006**
(21) Anmeldenummer: 04003175.9
(22) Anmeldetag: 12.02.2004
(51) Int. Cl.: C12C 12/00, C12C 7/20, C12C 7/26, A61P 29/00, A61K 31/00

(54) **Neues Brauverfahren, damit gebrautes Bier und dessen Verwendung**
New brewing process, beer brewed thereby and use thereof
Nouveau procédé de brasserie, bière ainsi brassée et son utilisation

(30) Priorität: 28.02.2003 DE 10308864
(43) Veröffentlichungstag der Anmeldung: 01.09.2004
(73) Patentinhaber: Paulaner Brauerei GmbH & Co. KG, 81541 München (DE)
(72) Erfinder: Hellich, Peter, Dr., 82031 Grünwald (DE); Winter, Peter, 81541 München (DE); Hippeli, Susanne, Dr., 86441 Zusmarshausen (DE); Elstner, Erich, F., Prof. Dr. rer. nat., 82194 Gröbenzell (DE); Dornisch, Kerstin, Dr., 83620 Feldkirchen (DE)
(74) Vertreter: Turi, Michael

(56) Entgegenhaltungen:
- EP-A- 0 361 165
- DE-C- 555 462
- US-A- 3 275 447
- US-A- 4 915 959
- US-A- 5 962 045

## Beschreibung

Die vorliegende Erfindung betrifft ein neues Brauverfahren, ein damit hergestelltes Bier und eine Verwendung des Bieres.

Die Einstellung gegenüber alkoholischen Getränken, vor allem solchen, die durch Gärung gewonnen werden, hat sich in den letzte Jahren geändert. Während früher von ärztlicher Seite zu einer vollkommen abstinenten Lebensführung geraten wurde, wird heute erkannt, daß ein gemäßigter Alkoholgenuß gesundheitsfördernd sein kann (ausgenommen davon sind natürlich Suchtkranke oder auch Leberkranke). Es hat sich in Studien herausgestellt, daß eine gemäßigte Menge an Alkohol die Gehirndurchblutung steigert und die Herzkranzgefäße erweitert. Wissenschaftliche Langzeituntersuchungen haben ergeben, daß maßvolle Konsumenten alkoholhaltiger Getränke weniger anfällig für Herzkrankheiten und Bluthochdruck sind. Überraschend hat sich herausgestellt, daß ein maßvoller Alkoholkonsum im Vergleich zu Alkoholabstinenz, aber auch exzessivem oder übermäßigem Alkoholkonsum zu einer längeren Lebenserwartung führt. Dies ist insbesondere darauf zurückzuführen, daß die maßvollen Alkoholtrinker deutlich weniger Herzinfarkte haben. Heute lassen sich diese epidemiologischen Befunde auch physiologisch deuten. Maßvoller Alkoholgenuß erhöht nämlich das High-Density-Lipoprotein (HDL), das als gefäßschützend gilt, da es Ablagerungen an den Gefäßwänden verzögern kann. Andere Ergebnisse zeigen, daß kleine Alkoholmengen der Bildung von Blutgerinnsel und Blutpfropfen entgegenwirken und zu einem deutlich geringeren Verschlußgrad von koronaren Arterien führen. Alkohol in mäßigen Mengen wirkt auch als Abfänger von freien Radikalen, die bekanntlich aufgrund ihrer sehr hohen chemischen Reaktivität neben einer erforderlichen immunologischen Wirkung auch äußerst unerwünschte biochemische und physiologische Folgen, einschließlich einer DNS-Schädigung, haben können. Durch hohe Alkoholmengen hingegen werden freie Radikale gebildet.

Es ist jedoch nicht allein der Alkoholgehalt, der gegorene alkoholische Getränke ins Blickfeld der Gesundheitsförderung gerückt hat. In Rotwein und Bier sind es insbesondere die Polyphenole, denen eine positive gesundheitliche Wirkung zugeschrieben wird, da sie freie Radikale, insbesondere Sauerstoffradikale, im Blut abfangen können.

Polyphenole kommen in Getreide in größerem Maß nur in den Arten Gerste und Hirse vor. Zu nennen sind beispielsweise Catechin, Epicatechin und Procyanidin B 3.

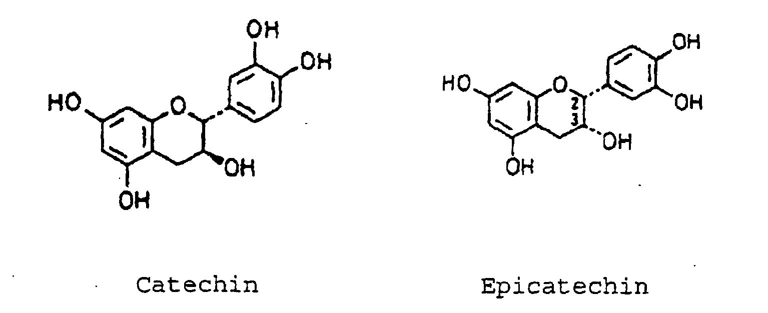

Auch Hopfen enthält Polyphenole. Das mengenmäßig und in seiner Wirkung bedeutendste ist das Prenylflavonoid Xanthohumol, das bei der Würzekochung im Brauverfahren zum größten Teil in Isoxanthohumol überführt wird.

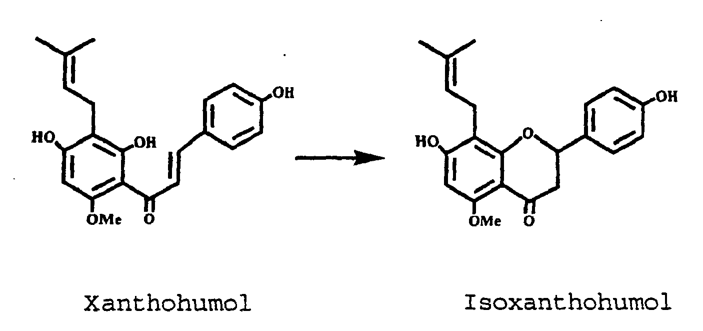

In biologischen Tests zeigt Isoxanthohumol ein hohes anticancerogenes Potential. Es hemmt beispielsweise die Wirkung des Enzyms Cytochrom P 450 CYPlA2, das Aflatoxin B1 in das cancerogene Aflatoxin M1 überführt.

Bier enthält auch eine oder mehrere antimutagen wirksame Substanzen. Im Tierversuch mit Mäusen wurde gezeigt, daß Bier die mutagene Wirkung bestimmter heterocyclischer Amine hemmt, die beispielsweise beim Braten von Fisch und Fleisch entstehen und im Zigarettenrauch vorliegen. Es ist noch nicht geklärt, um welche Verbindungen es sich handelt. Ein Experiment weist auf einen Beitrag von Hopfen hin. In einer Simulation der Würzekochung wurden 1,5 g Hopfen während 90 Minuten in 1 l Wasser bei pH 5 gekocht. Die resultierende wäßrige Lösung erwies sich als antimutagen aktiv.

Bier zeigt noch eine weitere überraschende Wirkung: es wurde festgestellt, daß Biertrinker (ebenso wie Weintrinker) weniger häufig mit Heliobacter pylori befallen sind als Abstinenzler. Dieses Bakterium gilt als Hauptverursacher von Magengeschwüren. Es wurde in der Tat nachgewiesen, daß ein spezieller Inhaltsstoff von Hopfen, nämlich Lupolon (eine sogenannte Beta-Säure) eine hohe Aktivität gegen Heliobacter pylori aufweist.

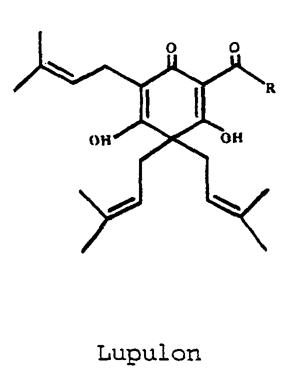

Hopfen wurde in der Pflanzenheilkunde bereits im Mittelalter u.a. wegen seiner antiseptischen Wirkung verwendet.

Bier ist das einzige Lebensmittel, das Hopfen enthält, und es ist zu vermuten, daß noch weitere pharmazeutisch wirksame Substanzen im Bier entdeckt werden.

Das Patent DE 555 462 C offenbart ein Verfahren zur Herstellung eines Stammbiers, das als überkonzentrierte, nicht normal schmeckende alkoholische Flüssigkeit auf dem Gärungsweg erhalten wird, die erst im Gebrauchsfall durch Verdünnen und Zusatz von Kohlensäure in eine trinkfertige Form überführt wird.

Das US-Patent US-A-3 275 447 beschreibt ein Verfahren für die Herstellung eines getrockneten Hopfenkonzentrats, das im Wesentlichen alle Lupulin-Komponenten oder Bitterstoffe des Hopfens enthält. Dabei wird der Hopfen auf eine Temperatur von mindestens -10°C eingefroren, wobei das Lupulin diskrete Teilchen bildet, der gefrorenen Hopfen mit Inertgas-Wirbelströmen auf eine Teilchengröße von 200 bis 6000 Mikrometern zerkleinert, wodurch die Lupulin-Teilchen aus dem Hopfen freigesetzt werden, der zerkleinerte Hopfen in eine grobe Fraktion, die kein Lupulin enthält, und eine feine Fraktion, die im wesentlichen alles Lupulin in dem Hopfen enthält, aufgeteilt und die feine Fraktion als Hopfen-Konzentrat gewonnen.

Im US-Patent US-A-4 915 959 wird Bier unter Verwendung von Hefe vergoren, danach wird die Hefe entfernt, und die Reifung des Biers wird durch eine Wärmebehandlung vorgenommen, um im wesentlichen alles alpha-Acetolacetat und andere Diacetyl-Vorstufen in Diacetyl zu überführen. Dann wird das Bier abgekühlt und durch eine Reaktionssäule geleitet, die mit immobilisierten Hefezellen gepackt ist, und zwar mit einer solchen Durchflußgeschwindigkeit, daß die Umwandlung des Diacetyls in Acetoin bewirkt wird, damit der Diacetyl-Gehalt auf ein geschmacksverträgliches Maß abgesenkt wird.

Die europäische Patentanmeldung EP 0 361 165 A1 offenbart die Produktion von Ethanol und spezieller von alkoholischen Getränken unter Verwendung von immobilisierter Hefe, die an ein regenerierbares Trägermaterial gebunden ist.

Die vorliegende Erfindung hat sich zur Aufgabe gestellt, weitere gesundheitsfördernde Wirkungen von Bier nachzuweisen und auf dieser Grundlage ein Bier zu brauen, das diese Wirkungen am effektivsten zeigt.

Diese Aufgabe wurde durch ein Brauverfahren gemäß Anspruch 1 und ein Bier gemäß Anspruch 8 gelöst.

Das erfindungsgemäße Brauverfahren ist dadurch gekennzeichnet, daß (a) dunkles Gerstenmalz in einer solchen Menge verwendet wird, daß sich ein Stammwürzegehalt von 12,5 bis 15 % (Gew./Gew.) ergibt, (b) zu Beginn des Würzekochens, das über 1 h 10 min bis 1 h 55 min durchgeführt wird, ein Ethanol-Extrakt von Hopfen in einer Menge, die 5,65 g bis 6,30 g α-Säure/hl Ausschlagwürze ergibt, und gegen Ende des Kochens Hopfenpellets Typ 90 in einer Menge, die 3,70 g bis 5,20 g α-Säure/hl Ausschlagwürze ergeben, zugesetzt werden, (c) das Bier nach der Nachgärung weder filtriert noch stabilisiert, sondern lediglich durch Zen-trifugieren geklärt wird, wodurch es eine Trübung (90°-Messung) in der Größenordnung von 20 EBC und eine Hefezellenzahl in der Größenordnung von 10⁶/ml aufweist.

Ein bevorzugtes Verfahrensmerkmal besteht weiter darin, daß die Hauptgärung drucklos bei 10°C bis 11°C, insbesondere 10,5°C, stattfindet. Weiter weist das Bier nach Nachgärung bevorzugt einen Alkoholgehalt von 5,5 bis 5,7 Vol.% Alkohol, insbesondere 5,59 Vol.% auf. Die Stammwürze beträgt in einer bevorzugten Ausführungsform 13,5 bis 14,0%, insbesondere 13,82% (Gew./Gew.). Die Würzekochzeit beträgt bevorzugt 1 Stunde 30 Minuten bis 1 Stunde 50 Minuten, insbesondere etwa 1 Stunde 40 Minuten. Das Maischverfahren wird bevorzugt als Zweimaischverfahren mit zwei Teilmaischen durchgeführt. Bevorzugt wird der Hopfen pro 670 hl Ausschlagmenge als Ethanol-Extrakt von Hallertauer Magnum (HMa)-Hopfen in einer 4000 g α-Säure enthaltenden Menge und zusätzlich als Tettnanger Tettnanger- (TTe)-Hopfenpellets Typ 90 in einer 3000 g α-Säure enthaltenden Menge zugesetzt.

Das durch dieses Verfahren erhältliche Bier wurde systematisch im Hinblick auf eine Optimierung einer neu entdeckten Eigenschaft von einigen kommerziellen und Testbieren entwickelt.

Durch systematische Untersuchung von in-vivo-Auswirkungen verschiedener Biere hat sich überraschend herausgestellt, daß einige Biersorten eine immunmodulatorische Wirkung besitzen. Genauer gesagt, wurde festgestellt, daß diese Biersorten zu einem gewissen Ausmaß eine entzündungsbedingte erhöhte Anzahl von neutrophilen Granulozyten und/oder eine entzündungsbedingte sehr hohe Aktivierbarkeit der neutrophilen Granulozyten, die eine "überschießende" Immunantwort dieser Leukozyten anzeigt, herunterregulieren konnten. Es wurde nun systematisch untersucht, welche Bestandteile des Bieres im Vollblut von Probanden mit erhöhter zahl an Neutrophilen und/oder erhöhter Aktivierbarkeit der Neutrophilen eine Herunterregulierung dieser beiden Parameter beeinflussen. Es wurde festgestellt, daß der wasserlösliche Anteil der Maische hauptverantwortlich war, daß aber auch ein saurer Hopfenpellet-Extrakt, wie er beim Würzekochen entsteht, einen Beitrag lieferte. Je höher diese beiden Anteile waren, desto ausgeprägter war die Immunmodulation.

Aufgrund dieser Befunde wurde das erfindungsgemäße Bier mit hohem Stammwürze- und Hopfengehalt konzipiert. Weiter wurde auf eine Filtration und Stabilisierung nach der Gärung verzichtet, um den Gehalt an Polyphenolen und anderen reduzierenden Substanzen nicht zu erniedrigen.

Nachstehend wird das erfindungsgemäße Brauverfahren und das erfindungsgemäße Bier anhand von nicht-beschränkenden Beispielen näher erläutert.

### Beispiel 1

Es wird eine Schüttung von 12 t Münchner Malz (Dunkles Gerstenmalz) verwendet. Das Gerstenmalz stammt ausschließlich aus zweizeiliger Sommerbraugerste der Sorten Scarlet, Annabell, Barke, Alexis, Pasadena, Hanka, Aspen, Danuta, Penelope, Prosa, Nevada und Optic mit mindestens 6tägiger Keimung.

Der Hauptguß besteht aus 410 hl enthärtetem Brauwasser mit 1 bis 2° deutscher Härte.

Es wird 20 Minuten bei 50°C im Maischbottich eingemaischt. Dann erfolgt das Erwärmen einer ersten Teilmaische mit 200 hl aus der Gesamtmaische, die dazu aus dem Maischbottich in die Maischpfanne überführt werden.

Die Temperatur der ersten Teilmaische wird innerhalb von 20 Minuten auf 65°C gebracht, dort 10 Minuten gehalten, dann innerhalb von weiteren 10 Minuten auf 73°C gebracht und dort mindestens solange gehalten, bis die Iod-Stärke-Reaktion negativ ist, was im vorliegenden Beispiel 15 Minuten sind. Anschließend wird eine Enderwärmung mit einem 15minütigen Anstieg der Temperatur auf 98°C und 10minütigem Kochen bei dieser Temperatur vorgenommen. Die erste Teilmaische wird dann in den Maischbottich zurückgeführt, dessen Inhalt sich dadurch auf 68°C erwärmt.

Nun wird eine zweite Teilmaische mit 120 hl aus der Maische im Maischbottich mit einer Temperatur von 68°C in die Maischpfanne überführt und dort innerhalb von 5 Minuten auf 73°C gebracht. Auch diese zweite Teilmaische wird 15 Minuten bei 73°C gehalten, dann innerhalb von 15 Minuten auf 98°C gebracht, dort 10 Minuten gekocht und anschließend in den Maischbottich zurückgeführt, wodurch die Temperatur der Gesamt-Maische auf 76°C ansteigt.

Die erhaltene Maische wird nun während ca. 4 bis 4,5 Stunden geläutert. Das Auslaugen des Trebers geschieht mit 350 hl Anschwänzwasser. Dies ergibt eine Pfanne-Voll-Würze (Läutermenge) von etwa 750 hl.

Nun erfolgt in der Sudpfanne das Würzekochen über 1 Stunde 42 Minuten unter Zugabe von Hopfen, die wie folgt vorgenommen wird:
- Zu Beginn des Würzekochens wird ein Ethanol-Extrakt von Hallertauer Magnum (HMa)-Hopfen in einer 4000 g α-Säure enthaltenden Menge zugesetzt (der Hopfenextrakt enthält ca. 40-50 Gew.% α-Säure);
- im weiteren Verlauf des Würzekochens werden Tettnanger Tettnanger- (TTe)-Hopfenpellets Typ 90 (Typ 90 bedeutet, daß die Pellets 90 Gew.% der Hopfendolde enthalten) in einer 3000 g α-Säure enthaltenden Menge zugesetzt, wobei die Zugabe kurz vor Ende des Würzekochens vorgenommen wird.

Die Menge der so gewonnenen Ausschlagwürze beträgt ca. 670 hl, die im Whirlpool einer weiteren Behandlung zur Entfernung von Trübstoffen (Heißtrub) unterzogen wird. Die Standzeit im Whirlpool beträgt 20 Minuten.

Anschließend wird die Ausschlagwürze im Würzekühler innerhalb von 60 Minuten auf 9-9,5 °C abgekühlt.

Die Hauptgärung erfolgt drucklos bei 10,5°C über 7 Tage, bis ein Restextrakt von 5,0% erreicht ist. Das Jungbier wird anschließend auf 6°C abgekühlt und in den Lagerkeller gepumpt, wo es unter Gegendruck von etwa 0,5 bar (auf 0,5 bar gespundet) bei 2°C nachgärt. Die Reifung (Nachgärung) und Lagerung dauert 6 Wochen.

Danach wird das Bier so zentrifugiert, daß sich eine Trübung von 20 EBC (European Brewery Convention) einstellt. Es erfolgt keine Filtration oder Stabilisierung des Bieres. Vor der Abfüllung wird der CO₂-Gehalt mittels Karbonisierungsanlage auf 0,57% eingestellt.

Zum Schluß erfolgt eine Abfüllung in Flaschen oder Fässer.

### Beispiel 2

Das gemäß Beispiel 1 erhaltene Bier weist die folgenden Merkmale auf:

| | |
|---|---|
| Stammwürze (Gew./Gew.): | 13,82% |
| Alkoholgehalt (Gew./Gew.) | 4,37% |
| Alkoholgehalt (Vol.%) | 5,59% |
| Wirklicher Extrakt (Gew./Gew.): | 5,45% |
| Scheinbarer Extrakt (Spindelmessung) (Gew./Gew.): | 3,46% |
| Wirklicher Vergärungsgrad | 62,3% |
| Scheinbarer Vergärungsgrad | 75,0% |
| Farbe (EBC)* | 39,2 |
| Bitterstoffe (Messung der W-Absorption in Isooctanlösung bei 275 nm) (BE)** | 33,1 |
| Schaum (s nach NIBEM)*** | 247 s |
| CO₂ | 0,57% |
| Trübung (90°-Messung) (EBC)**** | ca. 20 |
| Hefezellenzahl | ca. 10⁶/ml |

| | |
|---|---|
| * European Brewery Colours | |
| ** Bittereinheiten | |
| *** Gemäß MEBAK, Brautechnische Analysemethoden Band II, 4. Auflage (2002) | |
| **** Formazinstandardtrübung nach Vorschrift der European Brewery Convention (EBC) | |
| Alle angeführten Analysen wurden gemäß MEBAK, Brautechnische Analysenmethoden, Band II, 4. Auflage (2002) durchgeführt | |

Ein weiterer Gegenstand der Erfindung ist die Verwendung des Bieres von Anspruch 8 oder gebraut nach Anspruch 1 als Lebensmittel, das die Zahl und die Aktivität von neutrophilen Granulozyten herrunterregulieren kann.

Die biologischen Tests, welche dieser Verwendung zugrunde liegen, werden nachstehend näher erläutert.

Zum besseren Verständnis der Tests seien hier nochmals kurz die wichtigsten Funktionen neutrophiler Granulozyten beschrieben.

Polymorphkernige neutrophile Granulozyten, die einen Teil der Leukozyten (weiße Blutkörperchen) bilden, zirkulieren im Blut, dringen aber bei einem Entzündungsgeschehen in das betroffene Gewebe oder Organ ein und erfüllen dort ihre Aufgabe im Rahmen der Immunabwehr. Die Hauptfunktionen der Granulozyten sind einmal die Phagozytose (Aufnahme partikulären Materials in die Zelle) von eingedrungenen Pathogenen oder anderen Fremdkörpern, und weiter nach Aktivierung durch das Immunsystem die Erzeugung der reaktiven Sauerstoffspezies Superoxidradikalanion (O₂·⁻) und Wasserstoffperoxid (H₂O₂) (sogenannter "respiratory burst"; durch biochemische Folgereaktionen können daraus auch das OH-Radikal (OH·) und Singulettsauerstoff (¹O₂) entstehen) sowie die Freisetzung von Granula (Degranulation), wodurch u.a. das Enzym Myeloperoxidase (MPO) freigesetzt wird. MPO kann die Umsetzung von Wasserstoffperoxid mit Halogenidanionen (X⁻) zu Hypohalogenitanionen, im allgemeine Hypochloritanionen (OCl⁻), oder Hypochlorige (oder auch Unterchlorige) Säure (in saurem Milieu) katalysieren. Hypochlorige Säure kann mit dem Indikatormolekül 1-Aminocyclopropyl-1-carbonsäure (ACC) quantitativ nachgewiesen werden, da die Hypochlorige Säure mit ACC unter Bildung von Ethen reagiert, das gaschromatographisch sehr genau bestimmt werden kann.

Die von den Neutrophilen freigesetzten Substanzen können bei anhaltender Entzündung außer dem Fremdorganismus in einer überschießenden Reaktion auch das körpereigene Gewebe angreifen. Deshalb ist es sinnvoll, eine allzu hohe Zahl und Aktivität der Neutrophilen herunterzuregulieren.

### Testverfahren

Die Erfassung der Anzahl von neutrophilen Granulozyten im Blut wurde im Rahmen eines großen Blutbildes vorgenommen, das in einem medizinischen Labor erstellt wurde. Dabei ist eine Zahl bis zu 5000 Neutrophile in 1 µl Blut als normal anzusehen, während höhere Werte auf einen Entzündungsprozeß hinweisen.

Ein Maß für die Aktivität der neutrophilen Granulozyten ist deren Aktivierbarkeit durch Zugabe von Zymosan (einer Hefezellwandpräparation) zu Vollblut. Zymosan unterliegt im Vollblut sofort einer Opsonisierung mit Immunglobulin G und Komplementfaktoren und aktiviert dann die neutrophilen Granulozyten durch Wechselwirkung mit Oberflächerzeptoren derselben, wodurch der Phagocytoseprozeß, der "respiratory burst" und die Degranulation ausgelöst werden. Die dabei entstehende Hypochlorige Säure wird wie folgt mit ACC nachgewiesen:

### Allgemeines Testprotokoll

100 ml Probandenblut werden sofort mit 4 ml EDTA zur Vermeidung der Blutgerinnung versetzt (= Vollblut). Zu 1 ml Vollblut werden 0,1 ml ACC (2,5 mM), 0,5 ml einer wäßrigen Zymosan-Suspension (10 mg/ml) und PBS-Puffer ad 2 ml Gesamtvolumen gegeben. Die Reaktionsansätze werden 60 Minuten bei 37°C im Wasserbad in Volumen-geeichten, gasdicht verschlossenen Gefäßen inkubiert, und anschließend wird die aus ACC freigesetzt Ethenmenge, die sich oben im Gefäß angesammelt hat, gaschromatographisch quantifiziert (sogenannte "Head-Space-Technik"). Die freigesetzte Ethenmenge/10⁶ neutrophile Granulozyten, ist ein Maß für die deren Aktivierbarkeit. Als normal gelten 500 - 1000 pmol Ethen/10⁶ neutrophile Granulozyten.

### In-vivo-Trinkversuche mit 2 Bieresorten

In einer vorläufigen Trinkstudie nahmen 6 Probanden (Probanden A bis F, drei weibliche, drei männliche) jeweils 2 Biersorten zu sich.

### 1. Biersorten

Bei der einen Biersorte handelte es sich um normales helles Paulaner Bier.

Bei der anderen Biersorte handelte es sich um ein experimentelles Bier, das wie folgt hergestellt wurde:
a) Geschrotetes Braugetreide wird mit Wasser gemischt (Maische) und 10 Minuten gekocht, dann werden die festen Bestandteile abzentrifugiert und der Überstand wird lyophilisiert. 40 g des Lyophilisats werden in 500 ml helles Bier gegeben, was einer 60%igen Erhöhung der löslichen Maischeanteile des Bieres entspricht.
b) Dann werden 125 mg Hopfenextrakt in 500 µl Ethanol (96%ig) gelöst und dem oben in Schritt a) erhaltenen Bier zugesetzt.
c) Schließlich wurden 2,5 g Hopfenpellets in 125 ml 1:50 Essigessenz (in Anlehnung an den pH-Wert beim Würzekochen) suspendiert und 30 Minuten bei Raumtemperatur gerührt. Die festen Bestandteile werden abzentrifugiert, und der Überstand wird lyophilisiert. 75 mg des Lyophilisats werden dem in Schritt b) erhaltenen Bier zugesetzt.

Man erhält so ein Bier mit hoher Stammwürze und hohem Hopfengehalt.

### 2. Trinkversuch

An den zwei Tagen vor dem ersten Trinkversuch führten alle Probanden ein Ernährungsprotokoll (am zweiten Tag war die Diät festgelegt) und nahmen keinen Alkohol zu sich. Am ersten Testtag, an dem ebenfalls eine festgelegte Diät eingehalten wurde, tranken alle Probanden 500 ml normales helles Paulaner Bier.

Dann folgten wieder zwei Tage unter den gleichen Bedingungen wie vor dem ersten Testtag. Am zweiten Testtag nahmen alle 6 Probanden bei festgelegter Diät 500 ml des oben beschriebenen experimentellen Bieres zu sich.

### 3. Ergebnisse beim Proband A

Proband A litt während der Versuche an einer Erkältung. Bei ihm wurde eine überraschende Wirkung des obigen experimentellen Bieres festgestellt.

Wie bei den anderen Probanden wurde ihm zu den Zeitpunkten 0, 30, 60, 90, 120 und 180 Minuten nach Einnahme des hellen Bieres und des experimentellen Bieres Blut abgenommen, das routinemäßig auch auf die Aktivierbarkeit der neutrophilen Granulozyten mittels Zymosan überprüft wurde.

Dabei stellte sich heraus, daß das Blut des Probanden in Einklang mit seiner Erkältung zum Zeitpunkt 0 eine erhöhte Aktivierbarkeit der neutrophilen Granulozyten zeigte (1. Versuchstag; 2037 pol Ethen pro 10⁶ neutrophile Granulozyten, 2. Versuchstag 2249 pmol Ethen pro 10⁶ neutrophile Granulozyten).

Nach Einnahme des normalen hellen Paulaner Bieres veränderte sich die Aktivierbarkeit der neutrophilen Granulozyten praktisch nicht.

Nach Einnahme des obigen experimentellen Bieres jedoch nahm die Ethenfreisetzung zum Zeitpunkt t = 120 Minuten um 14% und zum Zeitpunkt t = 180 Minuten um 22% ab.

Dies bedeutet, daß die krankheitsbedingte Überaktivität der neutrophilen Granulozyten durch das experimentelle Bier herunterreguliert wurde, jedoch nicht so weit, daß die wichtige Immunfunktion dieser Leukozyten aufgehoben wurde.

Bei den gesunden Probanden war dieser Effekt nicht oder nicht in dem Ausmaß zu sehen.

### Ex-vivo-Experimente

In diesen Experimenten sollte ex vivo der Einfluß verschiedener Bierinhaltsstoffe auf die Aktivierbarkeit von neutrophilen Granulozyten überprüft werden, um festzustellen, ob und gegebenenfalls welche Inhaltsstoffe sich regulierend auf dieselbe auswirken.

### 1. Art und Gewinnung der Bierinhaltsstoffe

Bei den Bierinhaltsstoffen handelte es sich um Anstellwürze (die bereits abgekühlte Würze nach dem Kochen) eines hellen Bieres, Läuterwürze (die abgeläuterte Würze vor dem Würzekochen) eines hellen Bieres, den wasserlöslichen Teil von Hefe unter Vermehrungsbedingungen, fertiges helles Bier, einen Hopfen-Ethanolextrakt bei pH 10, eine Hopfen-Ethanolextrakt bei pH 4, die wasserlöslichen Anteile der Maische und einen wäßrigen Extrakt von Hopfenpellets. Diese Inhaltsstoffe wurden jeweils in Konzentrationen von 0,025, 0,1, 0,5 und 5% zu dem Vollblut gegeben.

Der wasserlösliche Anteil von Maische wurde durch Abzentrifugieren der festen Teile einer Maische gewonnen.

Die verwendete Hefe war eine Hefe-Bier-Gemisch, das nach der Gärung entnommen worden war; der in Bier lösliche ("wasserlösliche") Teil wurde durch Abzentrifugieren der Hefezellen gewonnen.

Der wasserlösliche Anteil der Hopfenpellets wurde erhalten, indem man diese mit einer Kugelmühle mahlte, in bidestilliertem Wasser aufschlämmte, 60 Minuten bei Raumtemperatur rührte und anschließend die wasserunlösliche Phase durch Zentrifugieren entfernte.

Die wasserlösliche Anteile von Hopfenextrakt bei pH 4 und pH 11 wurden durch Emulgieren des Hopenextrakts in bidestilliertem Wasser, das Acetatpuffer (pH 4)enthielt, bzw. bidestilliertem Wasser, das Carbonatpuffer (pH 10) enthielt, 60 minütiges Rühren bei 95°C und sorgfältiges Entfernen der an der Oberfläche abgeschiedenen lipophilen Verbindungen gewonnen.

Läuter- und Anstellwürze sowie das fertige helle Paulaner Bier wurden, wie erhalten, verwendet.

### 2. Testverfahren und Ergebnisse

Zunächst wurden an Vollblut von 4 Probanden Kontrollversuche gemäß dem oben beschriebenen Allgemeinen Testprotokoll durchgeführt.

Dann wurde dem Vollblut vor der Zugabe von Zymosan jeweils ein Bierinhaltsstoff bei variierenden Konzentrationen zugesetzt, wobei ansonsten gemäß dem Allgemeinen Testprotokoll verfahren wurde.

Es zeigte sich, daß die Läuter- und Anstellwürze, der wasserlösliche Teil der Hefe, die beiden Hopfenextrakt-Präparationen und das fertige helle Bier die Aktivierung der neutrophilen Granulozyten nicht beeinflussen, d.h., die Freisetzung von Ethen ist nach Aktivierung durch Zymosan im Vergleich zum Blindversuch nicht signifikant verändert.

### a) Test mit wäßrigem Maische-Extrakt

Es zeigte sich bei dem Kontrollversuch zu Beginn des ex-vivo-Tests, daß die neutrophilen Granulozyten der Probanden 1 und 4 zu einer Ethenfreisetzung von 5700 pmol Ethen/60 min (Proband 1) bzw. 7500 pmol Ethen/60 min (Proband 4) Anlaß gaben, was darauf hinwies, daß diese beiden Probanden an einer entzündlichen Krankheit litten.

Die Zugabe von 5% wäßrigem Maische-Extrakt zum Blut des Probanden 1 führte zu einer Erniedrigung der Ethenfreisetzung auf etwa 2000 pmol Ethen/ 60 min. Die Zugabe von 5% wäßrigem Maische-Extrakt zum Blut des Probanden 4 führte sogar zu einer völligen Unterdrückung der Ethenfreisetzung.

Die Ethenfreisetzung beim Blut der Probanden 2 und 3, das im Kontrollversuch keine erhöhte Aktivierbarkeit der neutrophilen Granulozyten zeigte, wurde durch den wäßrigen Maische-Extrakt nicht signifikant beeinflußt.

### b) Test mit wasserlöslichem Anteil von Hopfenpellets

Hier wurde das Vollblut des Probanden 4 untersucht. Der Kontrollversuch zu Beginn des Experiments ergab wiederum einen sehr hohen Wert der Ethenfreisetzung, nämlich etwa 4500 pmol Ethen/60 min.

Nach Zugabe von wasserlöslichem Hopfenpellet-Anteil mit einer Konzentration 0,025 und 0,05% sank der Wert auf etwa 3500 pmol Ethen/min, nach Zugabe von 0,1 und 0,5% des wäßrigen Hopfenpellet-Extrakts sogar auf etwa 2750 pmol Ethen/60 min. Allerdings stieg der Wert der Ethenfreisetzung nach Zugabe von wäßrigem Hopfenpellet-Extrakt in einer Konzentration von 5% wieder auf den Ausgangswert.

Auch hier wurden die Werte der gesunden Probanden nicht signifikant beeinflußt

### c) Schlußfolgerung

Aus den ex-vivo-Experimenten wurde gefolgert, daß eine Erhöhung der Stammwürze und ein erhöhter Anteil an wäßrigem Hopfenpellet-Extrakt sich auch in vivo günstig auf die Herunterregulierung von überaktivierten neutrophilen Granulozyten auswirken könnte.

Demgemäß wurde ein Bier mit erhöhter Stammwürze und erhöhter Hopfenpellet-Zugabe beim Würzekochen, nämlich das erfindungsgemäße Bier, gebraut und in einer Trinkstudie mit größerer Probandenzahl getestet.

### Beispiel 3

### Trinkstudie mit dem erfindungsgemäßen Bier

An dieser Trinkstudie nahmen 17 Probanden (Probanden A bis Q) teil, die über drei Tage jeden Abend 1,0 1 des erfindungsgemäßen Bieres tranken.

Die Auswirkung des Biergenusses nach 45 Minuten und nach 4 Tagen auf die Zahl der neutrophilen Granulozyten sowie auf deren Aktivierbarkeit im Vergleich zu den anfänglichen Kontrollwerten ist in der nachstehenden Tabelle 1 gezeigt.
Für die Bestimmung der Ethenfreisetzung wurde 1 ml Vollblut verwendet.

**Tabelle 1**

| **Proband** | **Neutrophilen-Aktivität [pmol Ethen/60 min]** | | | **Neutrophilen-Anzahl [n/µl Vollblut]** | | |
|---|---|---|---|---|---|---|
| | **t = 0** | **t = 45 min** | **t = 4 Tage** | **t = 0** | **t = 45 min** | **t = 4 Tage** |
| **A** | 5681 ± 470 | 5093 ± 447 | 1476 ± 94 | 4565 | 4611 | 3290 |
| **B** | 3268 t 66 | 3154 ± 126 | 1181 ± 134 | 3111 | 3672 | 2703 |
| **C** | 6046 ± 254 | 5125 ± 139 | 1319 ± 96 | 5796 | 5568 | 3422 |
| **D** | 4372 ± 113 | 4895 ± 285 | 2062 ± 148 | 4424 | 4590 | 3410 |
| **E** | 2670 ± 177 | 4071 ± 112 | 1137 ± 57 | 2538 | 3705 | 2392 |
| **F** | 4609 ± 167 | 4104 ± 439 | 1785 ± 123 | 4602 | 4446 | 3658 |
| **G** | 5216 ± 355 | 4530 ± 152 | 2070 ± 140 | 3111 | 3127 | 1824 |
| **H** | 6650 ± 161 | 5500 ± 128 | 3019 ± 273 | 6175 | 6204 | 4420 |
| **I** | 4153 ± 302 | 3303 ± 226 | 2465 ± 116 | 4088 | 4032 | 3894 |
| **J** | 2513 ± 180 | 2825 ± 52 | 1747 ± 161 | 2016 | 2244 | 1748 |
| **K** | 6142 ± 212 | 5849 ± 250 | 6571 ± 426 | 5016 | 4940 | 7215 |
| **L** | 5708 ± 268 | 6070 ± 326 | 5351 ± 32 | 3835 | 4352 | 4209 |
| **M** | 2377 ± 84 | 3839 ± 369 | 4058 ± 251 | 6120 | 6222 | 3660 |
| **N** | 4734 ± 523 | 7095 ± 140 | 4523 ± 389 | 5529 | 6240 | 4256 |
| **O** | 4125 ± 185 | 4920 ± 424 | 6825 ± 398 | 5060 | 4992 | 5220 |
| **P** | 5692 ± 553 | 5184 ± 96 | 4785 ± 191 | 4466 | 4860 | 3224 |
| **Q** | 3850 ± 193 | 4182 ± 269 | 2859 ± 111 | 3776 | 4140 | 2900 |

### 1. Zahl der neutrophilen Granulozyten

Aus der Tabelle ist ersichtlich, daß, außer bei den Probanden K, L und O, die Zahl der neutrophilen Granulozyten durch den Bierkonsum abgenommen hat, in der Regel auf etwa 3000 bis 4000 Zellen/µl Blut. Nur bei den Probanden G und J nahm sie auf einen Wert von etwas unter 2000 ab.

Das Blut der Probanden C, H, K, M, N und O wies im Kontrolltest eine über das normale Maß (> 5000 Zellen/µl Blut) erhöhte Zahl von neutrophilen Granulozyten auf, d.h., bei ihnen lag ein entzündlicher Zustand vor. Im Fall der Probanden C, H, M und N sank die Zahl dieser Zellen 4 Tage nach dem dreitägigen Biergenuß auf ein normales Maß unter 5000 Zellen/µl Blut. Im Fall des Probanden O erhöhte sich die Zahl der neutrophilen Granulozyten leicht, im Fall des Probanden,K deutlich, was auf eine Verschlimmerung des entzündlichen Zustands schließen läßt. Bei dem Probanden L, der keine erhöhte Zahl neutrophiler Granulozyten im Blut aufwies, nahm die Zahl dieser Zellen ebenfalls leicht zu.

Insgesamt läßt sich feststellen, daß das erfindungsgemäße Bier immunmodulatorisch wirkt, indem es die Zahl der neutrophilen Granulozyten senkt, jedoch nicht auf ein solches Maß, daß eine Unterfunktion dieser wichtigen Immunzellen vorliegt. Insbesondere wurde bei 4 von 6 Probanden mit einer entzündlichen Reaktion die Zahl der neutrophilen Granulozyten auf ein Normalmaß verringert, was einer Abmilderung der entzündlichen Reaktion entspricht.

### 2. Aktivierbarkeit der neutrophilen Granulozyten

Beim Kontrolltest wurde im Blut der Probanden C, H und K eine stark erhöhte Aktivierbarkeit der neutrophilen Granulozyten (Ethenfreisetzung über 6000 pmol Ethen/60 min festgestellt (diese Probanden wiesen auch eine erhöhte Zahl dieser Zellen auf, s.o.). Beim Probanden C wurde diese Aktivierbarkeit (wie auch schon die Zahl der neutrophilen Granulozyten) durch den Biergenuß nach 4 Tagen sehr deutlich auf etwa 1300 pmol Ethen/60 min erniedrigt und beim Probanden H auf etwa 3000 pmol Ethen/60 min. Lediglich beim Probanden K, bei dem auch schon die Zahl der neutrophilen Leukozyten nach 4 Tagen angestiegen war, erhöhte sich die Aktivierbarkeit dieser Zellen von etwa 6142 auf etwa 6571 pmol Ethen/60 min. Hier lag offensichtlich eine Verschlimmerung des entzündlichen Geschehens vor, die durch das Bier nicht verhindert werden konnte. Auch beim Probanden M, der ebenfalls eine erhöhte Zahl an neutrophilen Granulozyten aufwies (s. oben), aber im Kontrollversuch keine erhöhte Aktivierbarkeit dieser Zellen zeigte, nahm die Aktivierbarkeit nach 4 Tagen deutlich von 2377 pmol Ethen/60 min auf 4058 pmol Ethen/60 min zu, obwohl die Zahl der neutrophilen Granulozyten am Tag 4 abgenommen hatte.

Bei allen übrigen Probanden nahm die Aktivierbarkeit der neutrophilen Granulozyten zwischen Kontrolltest und Tag 4 ab, jedoch nie unter 1000 pmol Ethen/60 min, so daß die Funktionsfähigkeit dieser immunologisch wichtigen Leukozyten erhalten blieb.

### 3. Schlußfolgerung

Das erfindungsgemäße Bier ist ein Regulator der immunologisch wichtigen neutrophilen Granulozyten, sowohl bezüglich deren Zahl als auch deren Aktivierbarkeit. Wie oben erwähnt, kann eine Überfunktion dieser Zellen sowohl hinsichtlich ihrer Zahl als auch hinsichtlich ihrer Aktivierbarkeit körpereigenes Gewebe schädigen. Beide Parameter werden durch den Genuß des erfindungsgemäßen Bieres im Regelfall herunterreguliert, jedoch nicht unter ein physiologisch erforderliches Maß. Das erfindungsgemäße Bier weist demgemäß eine erwünschte immunmodulierende Eigenschaft auf.

## Patentansprüche

1. Brauverfahren, **dadurch gekennzeichnet, daß** (a) dunkles Gerstenmalz in einer solchen Menge verwendet wird, daß sich ein Stammwürzegehalt von 12,5 bis 15 % Gew./Gew. ergibt, (b) zu Beginn des Würzekochens, das über 1 h 10 min bis 1 h 55 min durchgeführt wird, ein Ethanol-Extrakt von Hopfen in einer Menge, die 5,65 g bis 6,30 g α-Säure/hl Ausschlagwürze ergibt, und gegen Ende des Kochens Hopfenpellets Typ 90 in einer Menge, die 3,70 g bis 5,20 g α-Säure/hl Ausschlagwürze ergeben, zugesetzt werden, (c) das Bier nach der Nachgärung weder filtriert noch stabilisiert, sondern lediglich durch Zentrifugieren geklärt wird, wodurch es eine Trübung (90°-Messung) in der Größenordnung von 20 EBC und eine Hefezellenzahl in der Größenordnung von 10⁶/ml aufweist.

2. Brauverfahren nach Anspruch 1, **dadurch gekennzeichnet, daß** in dem Verfahren eine Hauptgärung drucklos bei 10°C bis 11°C, insbesondere 10,5°C, stattfindet.

3. Brauverfahren nach Anspruch 1 oder 2, **dadurch gekennzeichnet, daß** in dem Verfahren die Gärung einschließlich Nachgärung so geführt wird, daß das Bier einen Alkoholgehalt von 5,5 bis 5,7 Vol.% Alkohol, insbesondere 5,59 Vol.%, aufweist

4. Brauverfahren nach einem der Ansprüche 1 bis 3, **dadurch gekennzeichnet, daß** die Stammwürze 13,5 bis 14,0%, insbesondere 13,82 % Gew./Gew. beträgt.

5. Brauverfahren nach einem der Ansprüche 1 bis 4, **dadurch gekennzeichnet, daß** die Würzekochzeit 1 Stunde 30 Minuten bis 1 Stunde 50 Minuten, insbesondere etwa 1 Stunde 40 Minuten beträgt.

6. Brauverfahren nach einem der Ansprüche 1 bis 5, **dadurch gekennzeichnet, daß** in dem Verfahren ein Maischverfahren als Zweimaischverfahren mit zwei Teilmaischen durchgeführt wird.

7. Brauverfahren nach einem der Ansprüche 1 bis 6, **dadurch gekennzeichnet, daß** einer Ausschlagmenge von 670 hl ein Ethanol-Extrakt von Hallertauer Magnum (HMa)-Hopfen in einer 4000 g α-Säure enthaltenden Menge zugesetzt wird und Tettnanger Tettnanger (TTe)-Hopfenpellets Typ 90 in einer 3000 g α-Säure enthaltenden Menge zugesetzt werden.

8. Bier, erhältlich nach dem Verfahren von einem der Ansprüche 1 bis 7.

9. Bier nach Anspruch 8, **gekennzeichnet durch** die folgenden Eigenschaften:
| | |
|---|---|
| Stammwürze (Gew./Gew.): | 13,82% |
| Alkoholgehalt (Gew./Gew.) | 4,37% |
| Alkoholgehalt (Vol.%) | 5,59% |
| Wirklicher Extrakt (Gew./Gew.): | 5,45% |
| Scheinbarer Extrakt | |
| (Spindelmessung) (Gew./Gew.): | 3,46% |
| Wirklicher Vergärungsgrad | 62,3% |
| Scheinbarer Vergärungsgrad | 75,0% |
| Farbe (EBC) | 39,2 |
| Bitterstoffe (Messung der UV-Absorption in Isooctanlösung bei 275 nm) (BE) | 33,1 |
| Schaum (s nach NIBEM) | 247 s |
| CO₂ | 0,57% |
| Trübung (90°-Messung) (EBC) | ca. 20 |
| Hefezellenzahl | ca. 10⁶/ml |

10. Verwendung eines Biers nach Anspruch 8 oder 9 für die Herstellung eines Medikaments zur Herunterregulierung der Zahl und Aktivität von neutrophilen Granulozyten.

## Claims

1. Brewing method
**characterised in that**
(a) dark barley malt is used in such a quantity that a content of original wort of 12.5 to 15 % w/w is obtained,
(b) at the start of the wort boiling, which is implemented over 1 h 10 min to 1 h 55 min, an ethanolic extract of hops is added in a quantity, which produces 5.65 g to 6.30 g α-acid/hl finished wort; and towards the end of the boiling, hop pellets type 90 are added in a quantity, which produces 3.70 g to 5.20 g α-acid/hl finished wort,
(c) following the after-fermentation, the beer is neither filtered nor stabilised, but only cleared by centrifugation to provide a turbidity (90° measurement) in the order of magnitude of 20 EBC and a yeast-cell count in the order of magnitude 10⁶/ml.

2. Brewing method according to claim 1,
**characterised in that**
a primary fermentation of the method takes place in a pressureless manner at 10°C to 11°C, especially at 10.5°C.

3. Brewing method according to claim 1 or 2,
**characterised in that**
the fermentation including after-fermentation of the method is implemented in such a manner that the beer has an alcohol content of 5.5 to 5.7% vol. alcohol, especially 5.59% vol.

4. Brewing method according to any one of claims 1 to 3,
**characterised in that**
the quantity of original wort is from 13.5 to 14.0% w/w, especially 13.82% w/w.

5. Brewing method according to any one of claims 1 to 4,
**characterised in that**
the wort boiling time is 1 hour 30 minutes to 1 hour 50 minutes, especially approximately 1 hour 40 minutes.

6. Brewing method according to any one of claims 1 to 5,
**characterised in that**
a mashing process of the method is implemented as a double mashing process with two separate mashes.

7. Brewing method according to any one of claims 1 to 6,
**characterised in that**
an ethanolic extract of Hallertauer Magnum (HMa) hops in a quantity containing 4000 g α-acid and Tettnanger Tettnanger (TTe) hop pellets type 90 in a quantity containing 3000 g α-acid are added to a quantity of finished wort of 670 hl.

8. Beer obtainable using the method according to any one of claims 1 to 7.

9. Beer according to claim 8,
**characterised by**
the following properties:
| | |
|---|---|
| Original wort (w/w) | 13.82% |
| Alcohol content (w/w) | 4.37% |
| Alcohol content (% vol) | 5.59% |
| Actual extract (w/w) | 5.45% |
| Apparent extract (spindle measurement) (w/w) | 3.46% |
| Actual degree of fermentation | 62.3% |
| Apparent degree of fermentation | 75.0% |
| Colour (EBC) | 39.2 |
| Bitter principles (measurement of UV absorption in iso-octane solution at 275 nm)(BE) | 33.1 |
| Head (s according to NIBEM) | 247 s |
| CO₂ | 0.57% |
| Turbidity (90° measurement) | |
| (EBC) | approx 20 |
| Yeast-cell count | approx 10⁶/ml. |

10. Use of a beer according to claim 8 or 9 for the manufacture of a medicinal agent for lowering the number and activity of neutrophil granulocytes.

## Revendications

1. Procédé de brasserie, **caractérisé en ce que** (a) du malt d'orge foncé est utilisé en une quantité telle que l'on ait une teneur en moût primitif de 12,5 à 15 % poids/poids, (b) qu'au début de la cuisson du moût qui dure de 1 h 10 minutes à 1 h 55 minutes, on obtienne un extrait éthanolique de houblon en une quantité qui donne 5,65 g à 6,30 g d'acide α/hl de moût définitif et que vers la fin de la cuisson, on ajoute des pellets de houblon de type 90 en une quantité qui donne 3,70 g à 5,20 g d'acide α/hl de moût définitif, (c) que la bière n'est ni filtrée ni stabilisée après la fermentation secondaire mais est juste éclaircie par centrifugation, présentant ainsi une turbidité (mesure à 90°) d'un ordre de grandeur de 20 EBC et un nombre de cellules de levure de l'ordre de 10⁶/ml.

2. Procédé de brasserie selon la revendication 1, **caractérisé en ce que**, dans le procédé, une fermentation principale a lieu sans pression, de 20° C à 11° C, en particulier à 10,5° C.

3. Procédé de brasserie selon la revendication 1 ou 2, **caractérisé en ce que** dans le procédé, la fermentation y compris la fermentation secondaire est réalisée de telle sorte que la bière présente une teneur en alcool de 5,5 à 5,7 % en volume d'alcool, en particulier 5,59 % en volume.

4. Procédé de brasserie selon l'une quelconque des revendications 1 à 3, **caractérisé en ce que** le moût primitif est de 13,5 à 14,0 %, en particulier 13,82 % en poids/poids.

5. Procédé de brasserie selon l'une quelconque des revendications 1 à 4, **caractérisé en ce que** le temps de cuisson du moût est de 1 heure 30 minutes à 1 heure 50 minutes, en particulier d'environ 1 heure 40 minutes.

6. Procédé de brasserie selon l'une quelconque des revendications 1 à 5, **caractérisé en ce que** dans le procédé, une méthode de brassage est réalisée en tant que deuxième procédé de brassage avec deux brassages partiels.

7. Procédé de brasserie selon l'une quelconque des revendications 1 à 6, **caractérisé en ce que** l'on ajoute à une quantité déterminante de 670 hl, un extrait d'éthanol de houblon Hallertauer Magnum (HMa) en une quantité contenant 4000 g d'acide α et des pellets de houblon Tettnanger Tettnanger (TTe) de type 90 en une quantité contenant 3000 g d'acide α.

8. Bière obtenue selon le procédé de l'une quelconque des revendications 1 à 7.

9. Bière selon la revendication 8, **caractérisée par** les caractéristiques suivantes :
| | |
|---|---|
| Moût primitif (poids/poids) : | 13,82 % |
| Teneur en alcool (poids/poids) : | 4,37 % |
| Teneur en alcool (% en vol.): | 5,59 % |
| Extrait réel (poids/poids): | 5,45 % |
| Extrait apparent (mesure par aréomètre) (poids/poids): | 3,46 % |
| Degré d'atténuation réel: | 62,3 % |
| Degré d'atténuation apparent: | 75,0 % |
| Colorant (EBC): | 39,2 |
| Substance amère (mesure de l'absorption UV dans une solution d'isooctane à 275 nm) (BE) : | 33,1 |
| Mousse (s selon NIBEM) : | 247 s |
| CO₂ : | 0,57 % |
| Turbidité (mesure à 90°) (EBC) : | env. 20 |
| Nombre de cellules de levure : | env. 10⁶/ml |

10. Utilisation d'une bière selon la revendication 8 ou 9 pour la fabrication d'un médicament pour la diminution du nombre et de l'activité des granulocytes neutrophiles.
